# EUROPEAN PATENT APPLICATION

(11) **EP 4 803 486 A1**
(43) Date of publication of application: **09.09.2026**
(21) Application number: 24885780.7
(22) Date of filing: 30.10.2024
(51) Int. Cl.: C01G 9/02, A61K 8/19, A61K 8/25, A61K 8/36, A61K 8/891, A61Q 17/00

(54) **ZINC OXIDE POWDERS, COMPOSITION FOR OIL-DISPERSIBLE COSMETIC, AND COSMETIC**

(30) Priority: 31.10.2023 JP 2023186515
(71) Applicant: Tayca Corporation, Osaka-shi, Osaka 551-0022 (JP)
(72) Inventor: MITO, Shunsuke, Osaka-shi, Osaka 551-0022 (JP); OOSAKI, Daisuke, Osaka-shi, Osaka 551-0022 (JP)
(74) Representative: Grünecker Patent- und Rechtsanwälte PartG mbB
(86) International application number: PCT/JP2024/038752
(87) International publication number: WO 2025/095013

(57) **Abstract**

To provide a zinc oxide powder capable of preparing a cosmetic that can exhibit excellent visible light transmittance and ultraviolet shielding performance when applied to skin, an oil-dispersible cosmetic composition containing the zinc oxide powder, and a cosmetic. The zinc oxide powder of the present invention is characterized in that D₅₀ of number distribution obtained from a transmission electron microscope image is 100 to 200 nm, the aspect ratio is 1.1 to 1.4, and the crystallite size obtained from an X-ray diffraction spectrum is 70 to 200 nm. The oil-dispersible cosmetic composition of the present invention is characterized in that the zinc oxide powder of the present invention is dispersed in an oil. The water-in-oil emulsion cosmetic and the oil-in-water emulsion cosmetic of the present invention are characterized in that the oil-dispersible cosmetic composition of the present invention is blended, or the zinc oxide powder of the present invention is blended. The powder cosmetic of the present invention is characterized in that the zinc oxide powder of the present invention is blended.

## Description

### TECHNICAL FIELD

The present invention relates to a zinc oxide powder capable of preparing a cosmetic that can exhibit excellent visible light transmittance and ultraviolet shielding performance when applied to skin, an oil-dispersible cosmetic composition using the zinc oxide powder, and a cosmetic.

### BACKGROUND ART

In various cosmetics such as sunscreens applied to skin, an inorganic powder such as zinc oxide may be blended in some cases.

In recent years, however, there has been a trend toward requiring the use of so-called non-nano zinc oxide having a particle size of 100 nm or more (e.g., Patent Document 1), due to concerns that when a cosmetic blended with zinc oxide powder having a small particle size is applied, fine zinc oxide powder may penetrate into the skin; and studies have also been conducted on methods for producing zinc oxide powder having a large particle size (e.g., Patent Document 2).

### PRIOR ART DOCUMENTS

### PATENT DOCUMENTS

Patent Document 1: JP-T-2019-517514
Patent Document 2: WO 2012/169611

### SUMMARY OF INVENTION

### PROBLEMS TO BE SOLVED BY THE INVENTION

However, in general, the ultraviolet shielding performance of zinc oxide powder is better as the particle size becomes smaller, and when using a particle size that can be referred to as non-nano zinc oxide, it is difficult to obtain a cosmetic having good ultraviolet shielding performance. For example, in the sunscreen composition described in Patent Document 1, bis-oxyhexyloxyphenol methoxyphenyl triazine having ultraviolet shielding performance is used together with non-nano zinc oxide to compensate for the ultraviolet shielding performance that is insufficient with non-nano zinc oxide alone.

In addition, as the particle size of zinc oxide powder increases, there is also a problem that the visible light transmittance when a cosmetic containing the powder is applied to skin decreases, causing, for example, whitening.

Therefore, for zinc oxide powder having a size that can be referred to as so-called non-nano zinc oxide, development of a technology is required that enables, when blended in a cosmetic and applied to skin, excellent visible light transmittance and ultraviolet shielding performance to be ensured.

The present invention has been made in view of the above circumstances, and an object thereof is to provide a zinc oxide powder capable of preparing a cosmetic that can exhibit excellent visible light transmittance and ultraviolet shielding performance when applied to skin, an oil-dispersible cosmetic composition containing the zinc oxide powder, and a cosmetic.

### MEANS FOR SOLVING THE PROBLEMS

The zinc oxide powder of the present invention is characterized in that D₅₀ of number distribution obtained from a transmission electron microscope image is 100 to 200 nm, the aspect ratio is 1.1 to 1.4, and the crystallite size obtained from an X-ray diffraction spectrum is 70 to 200 nm.

The oil-dispersible cosmetic composition of the present invention is characterized in that the zinc oxide powder of the present invention is dispersed in an oil.

The water-in-oil emulsion cosmetic of the present invention is characterized in that the oil-dispersible cosmetic composition of the present invention is blended, or the zinc oxide powder of the present invention is blended.

The oil-in-water emulsion cosmetic of the present invention is characterized in that the oil-dispersible cosmetic composition of the present invention is blended, or the zinc oxide powder of the present invention is blended.

The powder cosmetic of the present invention is characterized in that the zinc oxide powder of the present invention is blended.

### ADVANTAGEOUS EFFECTS OF INVENTION

According to the present invention, it is possible to provide a zinc oxide powder capable of preparing a cosmetic that can exhibit excellent visible light transmittance and ultraviolet shielding performance when applied to skin, and an oil-dispersible cosmetic composition and cosmetics (water-in-oil emulsion cosmetic, oil-in-water emulsion cosmetic, and powder cosmetic) containing the zinc oxide powder.

In the water-in-oil emulsion cosmetic and the oil-in-water emulsion cosmetic of the present invention, transparency can be increased, whitening when applied to skin can be suppressed, and excellent ultraviolet shielding performance can be exhibited. Furthermore, in the powder cosmetic of the present invention, whitening when applied to skin can be suppressed and excellent ultraviolet shielding performance can be exhibited.

### DETAILED DESCRIPTION OF EMBODIMENTS

### <Zinc Oxide Powder>

The zinc oxide powder of the present invention has D₅₀ of number distribution obtained from a transmission electron microscope (TEM) image (particle size at cumulative frequency 50% in terms of number basis; hereinafter sometimes simply referred to as "D₅₀") of 100 nm or more and 200 nm or less, an aspect ratio of 1.1 or more and 1.4 or less, and a crystallite size obtained from an X-ray diffraction spectrum (hereinafter sometimes simply referred to as "crystallite size") of 70 nm or more and 200 nm or less.

The zinc oxide powder of the present invention corresponds to so-called non-nano zinc oxide and has a D₅₀ of 100 nm or more. That is, "non-nano zinc oxide" as used in this specification means a zinc oxide powder having a D₅₀ of 100 nm or more. Zinc oxide of such a size, as described above, is inferior in ultraviolet shielding performance compared to zinc oxide of a smaller size, and also reduces visible light transmittance when made into a cosmetic, thereby tending to cause whitening when the cosmetic is applied to skin.

In the zinc oxide powder of the present invention, the upper limit value of D₅₀ is restricted and the aspect ratio and the crystallite size are set within specific ranges, thereby enabling, for example when blended in a cosmetic, the visible light transmittance and ultraviolet shielding performance when the cosmetic is applied to skin to be improved.

As described above, zinc oxide powder has a D₅₀ of 100 nm or more; however, if D₅₀ is too large, the visible light transmittance when the blended cosmetic is applied to skin decreases, and ultraviolet shielding performance also decreases. Therefore, from the viewpoint of enabling preparation of a cosmetic that can exhibit good visible light transmittance and ultraviolet shielding performance when applied to skin, D₅₀ of the zinc oxide powder is preferably 200 nm or less, more preferably 195 nm or less, and further preferably 190 nm or less.

Further, in the zinc oxide powder, D₁₀ of number distribution obtained by a TEM image (particle size at cumulative frequency 10% in terms of number basis; hereinafter sometimes simply referred to as "D₁₀") is preferably 100 nm or more and 200 nm or less, more preferably 100 nm or more and 160 nm or less, and further preferably 100 nm or more and 135 nm or less.

Further, in the zinc oxide powder, from the viewpoint of ensuring excellent ultraviolet shielding performance and achieving a good balance between this ultraviolet shielding performance and visible light transmittance when blended in a cosmetic and applied to skin, the aspect ratio is preferably 1.4 or less and 1.1 or more, and more preferably 1.2 or more.

Further, in the zinc oxide powder, if the crystallite size is too small, for example, the visible light transmittance when the blended cosmetic is applied to skin decreases. Therefore, from the viewpoint of increasing visible light transmittance when the blended cosmetic is applied to skin and improving the balance between this visible light transmittance and ultraviolet shielding performance, the crystallite size in the zinc oxide powder is 70 nm or more. However, if the crystallite size of the zinc oxide powder is too large, ultraviolet shielding performance decreases. Therefore, from the viewpoint of ensuring excellent ultraviolet shielding performance, and from the viewpoint of improving the balance between this ultraviolet shielding performance and visible light transmittance when the blended cosmetic is applied to skin, the crystallite size in the zinc oxide powder is preferably 200 nm or less, more preferably 180 nm or less, and further preferably 160 nm or less.

D₅₀, D₁₀, and the aspect ratio of the zinc oxide powder as used in this specification are values obtained by the following method. The zinc oxide powder is photographed by TEM. From the obtained image [number of particles (primary particles): 100], the particle size (particle size of the primary particle), major axis (major axis of the primary particle), minor axis (minor axis of the primary particle), and aspect ratio of each powder are calculated using image analysis particle size distribution measurement software. As a method for calculating particle size, the Heywood diameter (equivalent circle diameter based on projected area) is adopted. The major axis indicates the length of the long side of a rectangle circumscribing the selected particle when the area of the rectangle is minimized. The minor axis indicates the length of the short side of the rectangle circumscribing the selected particle when the area of the rectangle is minimized. D₅₀ and D₁₀ are calculated by the software based on the particle sizes of the obtained individual particles. The aspect ratio is the average value, over all particles (100 particles), of the aspect ratio calculated by the software for each particle as (major axis/minor axis).

The values described in Examples below were obtained using "JEM-1230" manufactured by JEOL Ltd. as the TEM and "Mac-View" manufactured by Mountech Co., Ltd. as the image analysis particle size distribution measurement software. D₉₀ of the number distribution of the zinc oxide powder described in Examples below (particle size at cumulative frequency 90% in terms of number basis; hereinafter sometimes simply referred to as "D₉₀") is also a value obtained by the same method as D₅₀ and the like.

The crystallite size of the zinc oxide powder as used in this specification is a value obtained by the following method. Using an X-ray diffractometer with Cu-Kα radiation, an X-ray diffraction spectrum of the zinc oxide powder is obtained under conditions of an X-ray output of 45 kV and 40 mA. The full width at half maximum of the peak near 36° (peak at 36.0±0.5°) in the obtained X-ray diffraction spectrum is measured, and the crystallite size of zinc oxide is calculated from the full width at half maximum using the Scherrer equation.

The values described in Examples below were obtained using "Xpert-PRO" manufactured by PANalytical as the X-ray diffractometer.

In the zinc oxide powder of the present invention, the ratio Tt550/Tt370 can be set to 3.5 or more, where Tt550 is the transmittance of light having a wavelength of 550 nm and Tt370 is the transmittance of light having a wavelength of 370 nm, each obtained using a spectral transmittance curve obtained from a film formed together with nitrocellulose (a film formed from the zinc oxide powder and nitrocellulose). Light having a wavelength of 370 nm corresponds to ultraviolet light, and light having a wavelength of 550 nm is in the visible light region. Accordingly, Tt550/Tt370 serves as an index of the balance between ultraviolet shielding performance and visible light transmittance in a cosmetic containing zinc oxide powder. When Tt550/Tt370 is 3.5 or more, for example, the balance between ultraviolet shielding performance and visible light transmittance in a cosmetic containing zinc oxide powder becomes better. The upper limit value of Tt550/Tt370 in the zinc oxide powder is not particularly limited, but is usually 8.0. If Tt550/Tt370 exceeds 8.0, there is a high possibility that it will no longer correspond to non-nano zinc oxide.

Further, Tt370, which is the transmittance of light having a wavelength of 370 nm obtained using a spectral transmittance curve obtained from a film formed from the zinc oxide powder and nitrocellulose, can be set to 24 or less, preferably 23 or less, and more preferably 22 or less. By setting Tt370 to these values, for example, better ultraviolet shielding performance can be ensured in a cosmetic containing zinc oxide powder. The lower limit value of Tt370 is not particularly limited, but is usually 10. If Tt370 is 10, it is possible to achieve SPF (Sun Protection Factor) 50 when the blending amount in a sunscreen is set to 25 mass%, which is the international upper limit for blending.

Further, Tt550, which is the transmittance of light having a wavelength of 550 nm obtained using a spectral transmittance curve obtained from a film formed from the zinc oxide powder and nitrocellulose, can be set to 80 or more, preferably 82 or more. By setting Tt550 to these values, for example, in a cosmetic containing zinc oxide powder, visible light transmittance can be increased to improve transparency and/or further suppress whitening when the cosmetic is applied to skin. The upper limit value of Tt550 is not particularly limited, but is usually 92. If Tt550 exceeds 92, there is a high possibility that it will no longer correspond to non-nano zinc oxide.

Tt370 and Tt550 as used in this specification are values obtained by the following method.

A film used for measurement is produced by the following method. First, nitrocellulose: 100.0 g, ethyl acetate: 140.0 g, n-butyl acetate: 210.0 g, ethylene glycol mono-n-butyl ether: 70.0 g, and toluene: 180.0 g are placed in a sealed container having a capacity of 1000 mL, and mixed for 12 hours at 100 rpm using a shaker to prepare a nitrocellulose solution. Next, the nitrocellulose solution: 40.0 g, zinc oxide powder: 1.0 g, and glass beads (φ1.5 mm): 50.0 g are placed in a sealed container having a capacity of 100 mL, and shaken to disperse for 1 hour at 1725 rpm using a test disperser to prepare a dispersion liquid of the zinc oxide powder.

The dispersion liquid of the zinc oxide powder is applied onto a polypropylene film and dried to obtain a film formed from zinc oxide and nitrocellulose. Total light transmittance of this film is measured using a spectrophotometer to obtain a spectral transmittance curve. From this spectral transmittance curve, the transmittance at a wavelength of 370 nm and the transmittance at a wavelength of 550 nm are read to obtain Tt370 and Tt550.

The values described in Examples below were obtained using the following materials and under the following conditions.
- Nitrocellulose: "Nitrocellulose (H1/2)" manufactured by Kishida Chemical Co., Ltd.
- Ethyl acetate, n-butyl acetate, ethylene glycol mono-n-butyl ether, toluene: manufactured by Sigma-Aldrich
- Sealed container: "J Bottle Round Wide Mouth 1000 mL" manufactured by NIKKO HANSEN; "J Bottle Round Wide Mouth 100 mL" manufactured by NIKKO HANSEN
- Shaker: "Thermo Shaker Z-1" manufactured by Thermonics
- Test disperser: "Paint Conditioner 1400" manufactured by Red Devil
- Polypropylene film: "Plain OPP Sheet #40" manufactured by Mitsui Chemicals Tohcello
- Spectrophotometer: "U-4100" manufactured by Hitachi High-Tech (using an integrating sphere)
   Measurement conditions by spectrophotometer
   Scan speed: 300 nm/min
   Sampling interval: 2 nm
   Measurement wavelength: 250 to 700 nm

In Examples below, the transmittance of light having a wavelength of 320 nm: Tt320 is also described; these values are obtained by the same method as Tt370 and the like.

With the zinc oxide powder described so far, it is possible to set Tt370, Tt550, and Tt550/Tt370 to the respective values described above.

The zinc oxide powder can be produced, for example, by a method in which an aqueous zinc chloride solution and an aqueous sodium carbonate solution are mixed to prepare a slurry containing basic zinc carbonate, basic zinc carbonate is taken out from this slurry, and fired.

The aqueous zinc chloride solution and aqueous sodium carbonate solution used for preparing the slurry containing basic zinc carbonate preferably have a low content of impurities (components other than zinc chloride and water in the aqueous zinc chloride solution, and components other than sodium carbonate and water in the aqueous sodium carbonate solution), whereby it becomes easy to adjust D₅₀ and D₁₀ of the finally obtained zinc oxide powder, as well as the aspect ratio and crystallite size, to the respective values described above.

The concentration of zinc chloride in the aqueous zinc chloride solution used for preparing the slurry containing basic zinc carbonate is, for example, 1 to 30 mass%. The concentration of sodium carbonate in the aqueous sodium carbonate solution used for preparing the slurry containing basic zinc carbonate is, for example, 1 to 20 mass%.

Conditions for firing the basic zinc carbonate taken out from the slurry include, for example, a firing temperature of 500 to 650°C and a firing time of 2 to 12 hours.

The zinc oxide powder may be surface-treated. Examples of a surface treatment agent for surface treatment of zinc oxide powder include at least one material (hydrophobizing agent) selected from the group consisting of silicone oil, fatty acid, and alkylsilane. Zinc oxide powder has hydrophilicity; however, by performing surface treatment using the above surface treatment agent functioning as a hydrophobizing agent, it becomes possible to increase hydrophobicity of the surface of the zinc oxide powder to, for example, improve dispersibility in oil used in cosmetics, and/or suppress the zinc oxide powder from falling off from the skin due to moisture such as sweat when the cosmetic is applied to skin.

Examples of silicone oil include so-called straight silicone oils such as dimethicone (dimethylpolysiloxane), phenyl dimethicone (methylphenylpolysiloxane), and hydrogen dimethicone (methylhydrogenpolysiloxane); and so-called branched silicone oils such as trimethylsiloxysilicic acid and triethoxysilylethyl polydimethylsiloxyethylhexyl dimethicone; and the like.

Examples of fatty acid include lauric acid, myristic acid, palmitic acid, stearic acid, isostearic acid, oleic acid, and behenic acid.

Examples of alkylsilane include alkoxysilanes such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, octyltriethoxysilane, and decyltrimethoxysilane; silazanes such as hexamethyldisilazane; and the like.

From the viewpoint of ensuring good effects of improving water resistance and improving hydrophobicity (improving dispersibility in oil) due to the use of the surface treatment agent, the content of the surface treatment agent in the zinc oxide powder is preferably 0.5 parts by mass or more, and more preferably 1 part by mass or more, per 100 parts by mass of the zinc oxide component in the powder. If the amount of the surface treatment agent in the zinc oxide powder is too large, for example, the amount of the zinc oxide component itself in the powder may become too small and the effects may become smaller. Therefore, the content of the surface treatment agent in the zinc oxide powder is preferably 30 parts by mass or less, and more preferably 25 parts by mass or less, per 100 parts by mass of the zinc oxide component in the powder.

Surface treatment using the above surface treatment agent can be performed by directly contacting zinc oxide powder with the surface treatment agent by mixing them, or by contacting zinc oxide powder with a solution in which the surface treatment agent is dissolved in an organic solvent (such as toluene) by mixing them.

### <Oil-Dispersible Cosmetic Composition>

The oil-dispersible cosmetic composition of the present invention is a composition used in cosmetics, in which the zinc oxide powder of the present invention and an oil are blended, with the zinc oxide powder being dispersed in the oil.

Oils that can be used in the oil-dispersible cosmetic composition are preferably those conventionally used in cosmetics applied to skin such as sunscreens, and include, for example, ester oils (liquid at 25°C), hydrocarbons (liquid at 25°C), and silicone oils (liquid at 25°C).

Examples of ester oil liquid at 25°C include isononyl isononanoate, glyceryl tri(caprylate/caprate), ethylhexyl palmitate, ethylhexyl methoxycinnamate, triethylhexanoin, cetyl ethylhexanoate, methylheptyl laurate, methylheptyl myristate, C12-15 alkyl benzoate, and the like.

Examples of hydrocarbon liquid at 25°C include hydrogenated polyisobutene, isododecane, mineral oil, squalane, and the like.

Examples of silicone oil liquid at 25°C include dimethicone, cyclopentasiloxane, cyclomethicone, diphenylsiloxy phenyl trimethicone, and the like.

The blending amount of the zinc oxide powder in the oil-dispersible cosmetic composition is not particularly limited, but is usually 20 to 70 mass%.

### <Water-in-Oil Emulsion Cosmetic and Oil-in-Water Emulsion Cosmetic>

The water-in-oil emulsion cosmetic and the oil-in-water emulsion cosmetic of the present invention are emulsion cosmetics in which the oil-dispersible cosmetic composition of the present invention is blended, or the zinc oxide powder of the present invention is blended. In preparing a water-in-oil emulsion cosmetic or an oil-in-water emulsion cosmetic, the zinc oxide powder of the present invention may be dispersed in an oil to prepare the oil-dispersible cosmetic composition of the present invention, and then a water-in-oil emulsion cosmetic or an oil-in-water emulsion cosmetic may be prepared using the composition. Alternatively, a water-in-oil emulsion cosmetic or an oil-in-water emulsion cosmetic may be prepared without performing a step of dispersing the zinc oxide powder in the oil.

When using zinc oxide powder that has been surface-treated using the above surface treatment agent (hydrophobizing agent), such zinc oxide powder usually has a high proportion existing in the oil phase in a water-in-oil emulsion cosmetic or an oil-in-water emulsion cosmetic due to high hydrophobicity of its surface. On the other hand, zinc oxide powder that has not been surface-treated has high hydrophilicity of its surface; therefore, when using such powder, the proportion of zinc oxide powder existing in the water phase in a water-in-oil emulsion cosmetic or an oil-in-water emulsion cosmetic tends to be high. To allow the zinc oxide powder to exist in the oil phase, a surfactant having a low HLB (Hydrophile-Lipophile Balance) may be used together.

The blending amount of the zinc oxide powder in the water-in-oil emulsion cosmetic and the oil-in-water emulsion cosmetic may be an amount required depending on the intended use and the like, but is usually 0.1 to 40 mass%.

Since the water-in-oil emulsion cosmetic and the oil-in-water emulsion cosmetic are emulsions, they contain water. The blending amount of water in the water-in-oil emulsion cosmetic and the oil-in-water emulsion cosmetic may be an amount required depending on the form (whether it is a water-in-oil emulsion or an oil-in-water emulsion, etc.) and intended use and the like, but is usually 10 to 80 mass%.

Further, since the water-in-oil emulsion cosmetic and the oil-in-water emulsion cosmetic are emulsions, they contain an oil. Examples of oils that can be used in the water-in-oil emulsion cosmetic and the oil-in-water emulsion cosmetic include those exemplified above as oils that can be blended in the oil-dispersible cosmetic composition. The blending amount of the oil in the water-in-oil emulsion cosmetic and the oil-in-water emulsion cosmetic may be an amount required depending on the form (whether it is a water-in-oil emulsion or an oil-in-water emulsion, etc.) and intended use and the like, but is usually 10 to 80 mass%.

As the water-in-oil emulsion cosmetic and the oil-in-water emulsion cosmetic a surfactant is usually blended to emulsify water and oil. As the surfactant, various surfactants (cationic surfactant, anionic surfactant, nonionic surfactant, amphoteric surfactant) commonly blended in ordinary cosmetics can be blended.

The blending amount of the surfactant in the water-in-oil emulsion cosmetic and the oil-in-water emulsion cosmetic may be an amount that can maintain a good emulsified state, for example, but is usually 1 to 10 mass%.

Further, in the water-in-oil emulsion cosmetic and the oil-in-water emulsion cosmetic, components required depending on the intended use and the like may be blended from among various components blended in ordinary cosmetics, . Examples of such components include fats and oils, higher alcohols, waxes, silicones, lower alcohols, ultraviolet absorbers, feel-improving agents, extender pigments, clay minerals, film-forming agents, thickeners, humectants (polyhydric alcohols and the like), preservatives, pH adjusters, fragrances, and the like.

### <Powder Cosmetic>

In the powder cosmetic of the present invention the zinc oxide powder of the present invention is blended. The blending amount of the zinc oxide powder in the powder cosmetic may be an amount required depending on the intended use and the like, but is usually 0.1 to 40 mass%.

In the powder cosmetic, components other than the zinc oxide powder may be blended. Examples of such components include inorganic powders other than zinc oxide powder, organic powders such as polymer powders, metal salt powders of surfactants (metal soaps), pigments, fragrances, preservatives, binders, and the like.

### EXAMPLES

Hereinafter, the present invention will be described in detail based on Examples. However, the Examples below are not intended to limit the present invention.

### <Preparation of Zinc Oxide Powder>

### Example 1

A 47 mass% aqueous zinc chloride solution: 132 g was diluted with ion-exchanged water: 519 g, and thereto was added a 10 mass% aqueous sodium carbonate solution: 430 g prepared by dissolving sodium carbonate: 43 g in ion-exchanged water: 387 g, to obtain a white slurry. The obtained slurry was filtered to collect a precipitate, and then the precipitate was washed with ion-exchanged water repeatedly until the electrical conductivity of the filtrated water became 150 µS/cm or less. The cake obtained by this operation was dried at 130°C for 12 hours and then fired at 500°C for 4 hours. The powder obtained by this firing was pulverized with a pin mill to obtain zinc oxide powder.

### Example 2

Zinc oxide powder was obtained in the same manner as in Example 1 except that the firing conditions after drying the cake were changed to 500°C for 6 hours.

### Example 3

Zinc oxide powder was obtained in the same manner as in Example 1 except that the firing conditions after drying the cake were changed to 550°C for 6 hours.

### Example 4

Zinc oxide powder was obtained in the same manner as in Example 1 except that the firing conditions after drying the cake were changed to 600°C for 2 hours.

### Comparative Example 1

Zinc oxide powder was obtained in the same manner as in Example 1 except that the firing conditions after drying the cake were changed to 600°C for 6 hours.

### Comparative Example 2

A 47 mass% aqueous zinc chloride solution: 132 g was diluted with ion-exchanged water: 396 g, and thereto was added a 10 mass% aqueous ammonia solution: 207 g to generate zinc oxide nuclei; subsequently, a 10 mass% aqueous sodium hydroxide solution: 352 g was added to the slurry to obtain a white slurry. Subsequently, while stirring the slurry, the temperature was raised to 90°C over 60 minutes, and then the slurry was heated and aged at 90°C for 30 minutes while stirring. After heating/aging, the obtained slurry was filtered to collect a precipitate, and then the precipitate was washed with ion-exchanged water repeatedly until the electrical conductivity of the filtrated water became 150 µS/cm or less. The cake obtained by this operation was dried at 130°C for 12 hours and then fired at 600°C for 2 hours. The powder obtained by this firing was pulverized with a pin mill to obtain zinc oxide powder.

### Comparative Example 3

Fine zinc oxide "MZ-150" (primary particle diameter: 80 nm) manufactured by Teika: 16.28 g was dispersed in ion-exchanged water: 500 g to obtain a zinc oxide slurry. Separately, hydrogen peroxide solution (manufactured by Wako Pure Chemical Industries, Ltd.): 20.77 g was diluted with ion-exchanged water: 500 g to prepare a hydrogen peroxide aqueous solution. Subsequently, while stirring the slurry, the hydrogen peroxide aqueous solution was added thereto, and the mixture was stirred at 25°C for 6 hours. Thereafter, the obtained liquid was filtered to collect a precipitate in the liquid and washed with water. The cake obtained by this operation was dried at 110°C for 12 hours and then fired at 600°C for 2 hours. The powder obtained by this firing was pulverized with a pin mill to obtain zinc oxide powder.

Physical property values of the zinc oxide powders of Examples and Comparative Examples are shown in Table 1.

Further, for the zinc oxide powders of Examples and Comparative Examples, Tt320, Tt370, and Tt550 were obtained by the method described above, and Tt550/Tt370 was calculated. These results are shown in Table 2.

Further, the specific surface areas of the zinc oxide powders of Examples and Comparative Examples were measured by the BET method using an automatic specific surface area measuring device ("Macsorb HM model-1208" manufactured by Mountech Co., Ltd.). Note that the degassing step during measurement was performed under conditions of 150°C for 20 minutes. These results are also shown in Table 1.

**[Table 1]**

| | D₁₀ (nm) | D₅₀ (nm) | D₉₀ (nm) | Aspect ratio | crystallite size (nm) | Specific surface area (m²/g) |
|---|---|---|---|---|---|---|
| Example 1 | 75 | 107 | 145 | 1.3 | 76 | 10 |
| Example 2 | 80 | 111 | 148 | 1.3 | 80 | 8 |
| Example 3 | 101 | 143 | 195 | 1.3 | 107 | 6 |
| Example 4 | 129 | 187 | 292 | 1.3 | 129 | 4 |
| Comparative Example 1 | 140 | 220 | 321 | 1.3 | 151 | 3 |
| Comparative Example 2 | 67 | 113 | 181 | 1.5 | 218 | 3 |
| Comparative Example 3 | 71 | 102 | 140 | 1.1 | 45 | 10 |

**[Table 2]**

| | Tt320 | Tt370 | Tt550 | Tt550/Tt370 |
|---|---|---|---|---|
| Example 1 | 21.6 | 13.7 | 90.1 | 6.58 |
| Example 2 | 19.6 | 12.2 | 89.2 | 7.31 |
| Example 3 | 23.2 | 17.1 | 86.3 | 5.05 |
| Example 4 | 28.7 | 21.0 | 84.4 | 4.02 |
| Comparative Example 1 | 35.3 | 25.2 | 73.2 | 2.90 |
| Comparative Example 2 | 34.2 | 28.0 | 88.7 | 3.17 |
| Comparative Example 3 | 26.7 | 19.2 | 76.1 | 3.96 |

As shown in Tables 1 and 2, the zinc oxide powders of Examples 1 to 4 in which all of D₅₀, aspect ratio, and crystallite size have appropriate values had a low Tt370 value, a high Tt550 value, and also a high Tt550/Tt370 value, thereby ensuring ultraviolet shielding performance and visible light transmittance (ultraviolet shielding performance and visible light transmittance when blended in a cosmetic and the like; the same applies hereinafter regarding ultraviolet shielding performance and visible light transmittance of zinc oxide powder) at a high level in a well-balanced manner. Note that the zinc oxide powder of Example 4 having relatively large D₅₀ and crystallite size had a higher Tt370 value and slightly inferior ultraviolet shielding performance compared to the zinc oxide powders of Examples 1 to 3, but practical-level properties could be ensured.

In contrast, the zinc oxide powder of Comparative Example 1 having too large D₅₀ had a high Tt370 value, a low Tt550 value, and also a low Tt550/Tt370 value, thereby being inferior in both ultraviolet shielding performance and visible light transmittance. Further, the zinc oxide powder of Comparative Example 2 having too large aspect ratio and crystallite size had a high Tt370 value and a low Tt550/Tt370 value, thereby being inferior in ultraviolet shielding performance and having a poor balance between ultraviolet shielding performance and visible light transmittance. Furthermore, the zinc oxide powder of Comparative Example 3 having too small crystallite size had a low Tt550 value and was inferior in visible light transmittance.

From the above results, it can be understood that the zinc oxide powders of Examples 1 to 4 can constitute a water-in-oil emulsion cosmetic and an oil-in-water emulsion cosmetic that are excellent in ultraviolet shielding performance, have relatively high transparency, and can suppress whitening when applied to skin, and can also constitute a powder cosmetic that is excellent in ultraviolet shielding performance and can suppress whitening when applied to skin.

### <Preparation of Cosmetics>

### (Preparation of Surface-Treated Zinc Oxide Powder)

### Example 5

Surface treatment was performed by contacting the zinc oxide powder of Example 1 with octyltriethoxysilane to prepare a surface-treated zinc oxide powder of Example 5.

### Comparative Example 4

A surface-treated zinc oxide powder was prepared in the same manner as in Example 5 except that the zinc oxide powder of Comparative Example 1 was used.

### Comparative Example 5

A surface-treated zinc oxide powder was prepared in the same manner as in Example 5 except that the zinc oxide powder of Comparative Example 2 was used.

### Comparative Example 6

A surface-treated zinc oxide powder was prepared in the same manner as in Example 5 except that the zinc oxide powder of Comparative Example 3 was used.

### (Preparation of Water-in-Oil Emulsion Cosmetic)

### Examples 6 to 8 and Comparative Examples 7 to 9

Water-in-oil emulsion cosmetics of Examples 6 to 8 and Comparative Examples 7 to 9 were prepared by blending the respective components shown in Table 3 in the proportions shown in Table 3 (each of the cosmetics after preparation was a water-in-oil emulsion).

For the water-in-oil emulsion cosmetics of Examples 6 to 8 and Comparative Examples 7 to 9, evaluation of ultraviolet shielding performance (ultraviolet protection effect) (in vitro test) was performed. The evaluation was performed based on an SPF measurement method (ISO 24443). Each of the water-in-oil emulsion cosmetics of Examples and Comparative Examples was applied in an amount of 1.3 mg/cm² onto an evaluation plate ["HELIOPLATE (registered trademark) HD6" manufactured by Helioscreen], and dried at room temperature for 30 minutes to prepare a measurement sample. Thereafter, SPF and UVAPF (Ultraviolet A Protection Factor) were measured using an SPF analyzer ("UV-2000S" manufactured by Labsphere). Further, from a spectral transmittance curve obtained during the SPF measurement, transmittance at a wavelength of 450 nm was obtained, and transparency of each water-in-oil emulsion cosmetic was also evaluated.

These results are also shown in Table 3. In Table 3, blending amounts of the respective components are shown in % such that the total of the cosmetic is 100%; each % is mass%, and in the table, the % symbol is omitted and only numerical values representing blending amounts are shown (the same applies to Tables 4 to 7 below). Further, "titanium oxide powder (A)" shown in Table 3 is "MT-N1" manufactured by Teika, and "spherical silica powder" is "TMS-T05DCB" manufactured by Teika (the same applies in Tables 5 to 7 below).

**[Table 3]**

| | Exampl e 6 | Exampl e 7 | Exampl e 8 | Comparati ve Example 7 | Comparati ve Example 8 | Comparati ve Example 9 |
|---|---|---|---|---|---|---|
| Oil phase | | | | | | |
| Surface-treated zinc oxide powder of Example 5 | 24.00 | 18.00 | - | - | - | - |
| Zinc oxide powder of Example 1 | - | - | 24.00 | - | - | - |
| Surface-treated zinc oxide powder of Comparative Example 4 | - | - | - | 24.00 | - | - |
| Surface-treated zinc oxide powder of Comparative Example 5 | - | - | - | - | 24.00 | - |
| Surface-treated zinc oxide powder of Comparative Example 6 | | - | - | - | - | 24.00 |
| Titanium oxide powder (A) | - | 4.00 | - | - | - | - |
| Octocrylene | - | - | 3.00 | - | - | - |
| Spherical silica powder | 5.00 | 5.00 | 5.00 | 5.00 | 5.00 | 5.00 |
| Sorbitan sesquiisostearate | 5.40 | 5.40 | 5.40 | 5.40 | 5.40 | 5.40 |
| Isopropyl palmitate | 24.10 | 29.00 | 17.60 | 24.10 | 24.10 | 24.10 |
| Glyceryl tri(caprylate/caprate) | 10.00 | 10.00 | 10.00 | 10.00 | 10.00 | 10.00 |
| Dextrin (palmitate/ethylhexano ate) | 1.50 | 1.50 | 2.00 | 1.50 | 1.50 | 1.50 |
| Polyhydroxystearic acid | - | - | 1.00 | - | - | - |
| Aqueous phase | | | | | | |
| Water | 24.90 | 22.00 | 22.00 | 24.90 | 24.90 | 24.90 |
| Sodium chloride | 2.00 | 2.00 | 2.00 | 2.00 | 2.00 | 2.00 |
| Methylparaben | 0.10 | 0.10 | - | 0.10 | 0.10 | 0.10 |
| Propanediol | - | - | 4.00 | - | - | - |
| Butylene glycol | 3.00 | 3.00 | 4.00 | 3.00 | 3.00 | 3.00 |
| Evaluation | | | | | | |
| SPF | 33 | 36 | 88 | 22 | 21 | 19 |
| UVAPF | 25 | 27 | 38 | 21 | 15 | 12 |
| Transmittance at wavelength 450 nm (%) | 47 | 53 | 41 | 27 | 44 | 31 |

As shown in Table 3, the water-in-oil emulsion cosmetics of Examples 6 to 8 had higher SPF and UVAPF values and excellent ultraviolet shielding performance compared to the cosmetics of Comparative Examples 7 to 9. Further, the water-in-oil emulsion cosmetics of Examples 6 to 8 each had a relatively high transmittance at a wavelength of 450 nm and also had excellent transparency.

### (Preparation of Oil-in-Water Emulsion Cosmetic)

### Examples 9 to 11 and Comparative Examples 10 to 18

Oil-in-water emulsion cosmetics of Examples 9 to 11 and Comparative Examples 10 to 18 were prepared by blending the respective components shown in Tables 4 to 6 in the proportions shown in Tables 4 to 6 (each of the cosmetics after preparation was an oil-in-water emulsion).

For the oil-in-water emulsion cosmetics of Examples 9 to 11 and Comparative Examples 10 to 18, evaluation of ultraviolet shielding performance (ultraviolet protection effect) and evaluation of transparency were performed in the same manner as for the water-in-oil emulsion cosmetic of Example 6 and the like. These results are also shown in Tables 4 to 6.

**[Table 4]**

| | Example 9 | Comparative Example 10 | Comparative Example 11 | Comparative Example 12 |
|---|---|---|---|---|
| Aqueous phase | | | | |
| Polyglyceryl-10 oleate | 2.00 | 2.00 | 2.00 | 2.00 |
| Butylene glycol | 5.00 | 5.00 | 5.00 | 5.00 |
| Water | 34.60 | 34.60 | 34.60 | 34.60 |
| Propanediol | 5.00 | 5.00 | 5.00 | 5.00 |
| (Hydroxyethyl acrylate/sodium acryloyldimethyl taurate) copolymer | 0.30 | 0.30 | 0.30 | 0.30 |
| Xanthan gum | 0.10 | 0.10 | 0.10 | 0.10 |
| Oil phase | | | | |
| Polyhydroxystearic acid | 1.00 | 1.00 | 1.00 | 1.00 |
| Isopropyl palmitate | 28.00 | 28.00 | 28.00 | 28.00 |
| Surface-treated zinc oxide powder of Example 5 | 24.00 | - | - | - |
| Surface-treated zinc oxide powder of Comparative Example 4 | - | 24.00 | - | - |
| Surface-treated zinc oxide powder of Comparative Example 5 | - | - | 24.00 | - |
| Surface-treated zinc oxide powder of Comparative Example 6 | - | - | - | 24.00 |
| Evaluation | | | | |
| SPF | 28 | 19 | 18 | 16 |
| UVAPF | 24 | 18 | 13 | 12 |
| Transmittance at wavelength 450 nm (%) | 68 | 50 | 64 | 52 |

**[Table 5]**

| | Example 10 | Comparative Example 13 | Comparative Example 14 | Comparative Example 15 |
|---|---|---|---|---|
| Aqueous phase | | | | |
| Polyglyceryl-10 oleate | 2.00 | 2.00 | 2.00 | 2.00 |
| Butylene glycol | 5.00 | 5.00 | 5.00 | 5.00 |
| Water | 36.70 | 36.70 | 36.70 | 36.70 |
| Propanediol | 5.00 | 5.00 | 5.00 | 5.00 |
| (Hydroxyethyl acrylate/sodium acryloyldimethyl taurate) copolymer | 0.20 | 0.20 | 0.20 | 0.20 |
| Xanthan gum | 0.10 | 0.10 | 0.10 | 0.10 |
| Oil phase | | | | |
| Polyhydroxystearic acid | 1.00 | 1.00 | 1.00 | 1.00 |
| Isopropyl palmitate | 24.00 | 24.00 | 24.00 | 24.00 |
| Surface-treated zinc oxide powder of Example 5 | 24.00 | - | - | - |
| Surface-treated zinc oxide powder of Comparative Example 4 | - | 24.00 | - | - |
| Surface-treated zinc oxide powder of Comparative Example 5 | - | - | 24.00 | - |
| Surface-treated zinc oxide powder of Comparative Example 6 | - | - | - | 24.00 |
| Titanium oxide powder (A) | 2.00 | 2.00 | 2.00 | 2.00 |
| Evaluation | | | | |
| SPF | 35 | 31 | 29 | 25 |
| UVAPF | 25 | 19 | 14 | 14 |
| Transmittance at wavelength 450 nm (%) | 66 | 52 | 63 | 54 |

**[Table 6]**

| | Example 11 | Comparative Example 16 | Comparative Example 17 | Comparative Example 18 |
|---|---|---|---|---|
| Aqueous phase | | | | |
| Polyglyceryl-10 oleate | 2.00 | 2.00 | 2.00 | 2.00 |
| Butylene glycol | 5.00 | 5.00 | 5.00 | 5.00 |
| Water | 40.00 | 40.00 | 40.00 | 40.00 |
| Propanediol | 5.00 | 5.00 | 5.00 | 5.00 |
| (Hydroxyethyl acrylate/sodium acryloyldimethyl taurate) copolymer | 0.40 | 0.40 | 0.40 | 0.40 |
| Xanthan gum | 0.10 | 0.10 | 0.10 | 0.10 |
| Oil phase | | | | |
| Polyhydroxystearic acid | 1.00 | 1.00 | 1.00 | 1.00 |
| Octocrylene | 5.00 | 5.00 | 5.00 | 5.00 |
| Bis-ethylhexyloxyphenol methoxyphenyl triazine | 1.50 | 1.50 | 1.50 | 1.50 |
| Isopropyl palmitate | 24.00 | 24.00 | 24.00 | 24.00 |
| Surface-treated zinc oxide powder of Example 5 | 12.00 | - | - | - |
| Surface-treated zinc oxide powder of Comparative Example 4 | - | 12.00 | - | - |
| Surface-treated zinc oxide powder of Comparative Example 5 | - | - | 12.00 | - |
| Surface-treated zinc oxide powder of Comparative Example 6 | - | - | - | 12.00 |
| Titanium oxide powder (A) | 4.00 | 4.00 | 4.00 | 4.00 |
| Evaluation | | | | |
| SPF | 54 | 43 | 42 | 37 |
| UVAPF | 12 | 8 | 8 | 7 |
| Transmittance at wavelength 450 nm (%) | 78 | 61 | 73 | 63 |

As shown in Table 4, the oil-in-water emulsion cosmetic of Example 9 had a higher SPF value, excellent ultraviolet shielding performance, a high transmittance at a wavelength of 450 nm, and excellent transparency compared to the cosmetics of Comparative Examples 10 to 12 having the same component composition except for the surface-treated zinc oxide powder.

As shown in Table 5, the oil-in-water emulsion cosmetic of Example 10 had a higher SPF value, excellent ultraviolet shielding performance, a high transmittance at a wavelength of 450 nm, and excellent transparency compared to the cosmetics of Comparative Examples 13 to 15 having the same component composition except for the surface-treated zinc oxide powder.

As shown in Table 6, the oil-in-water emulsion cosmetic of Example 11 had a higher SPF value, excellent ultraviolet shielding performance, a high transmittance at a wavelength of 450 nm, and excellent transparency compared to the cosmetics of Comparative Examples 16 to 18 having the same component composition except for the surface-treated zinc oxide powder.

### (Preparation of Powder Cosmetic)

Powder cosmetics of Example 12 and Comparative Examples 19 to 21 were prepared by blending the respective components shown in Table 7 in the proportions shown in Table 7.

For the powder cosmetics of Example 12 and Comparative Examples 19 to 21, evaluation of ultraviolet shielding performance (ultraviolet protection effect) was performed in the same manner as for the water-in-oil emulsion cosmetic of Example 6 and the like. These results are also shown in Table 7. "Titanium oxide powder (B)" shown in Table 7 is "MPY-1133M" manufactured by Teika, and "titanium oxide powder (C)" is "MT-100TV" manufactured by Teika.

**[Table 7]**

| | Example 12 | Comparative Example 19 | Comparative Example 20 | Comparative Example 21 |
|---|---|---|---|---|
| Solid phase | | | | |
| Yellow iron oxide | 1.50 | 1.50 | 1.50 | 1.50 |
| Red iron oxide | 0.40 | 0.40 | 0.40 | 0.40 |
| Black iron oxide | 0.15 | 0.15 | 0.15 | 0.15 |
| Titanium oxide powder (B) | 4.00 | 4.00 | 4.00 | 4.00 |
| Titanium oxide powder (C) | 7.00 | 7.00 | 7.00 | 7.00 |
| Surface-treated zinc oxide powder of Example 5 | 16.00 | - | - | - |
| Surface-treated zinc oxide powder of Comparative Example 4 | - | 16.00 | - | - |
| Surface-treated zinc oxide powder of Comparative Example 5 | - | - | 16.00 | - |
| Surface-treated zinc oxide powder of Comparative Example 6 | - | - | - | 16.00 |
| Spherical silica powder | 20.00 | 20.00 | 20.00 | 20.00 |
| Sericite | 8.00 | 8.00 | 8.00 | 8.00 |
| Synthetic fluorphlogopite | 19.95 | 19.95 | 19.95 | 19.95 |
| Boron nitride | 5.00 | 5.00 | 5.00 | 5.00 |
| Calcium silicate | 0.50 | 0.50 | 0.50 | 0.50 |
| Zinc myristate | 1.50 | 1.50 | 1.50 | 1.50 |
| Lauroyl lysine | 8.00 | 8.00 | 8.00 | 8.00 |
| Oil phase | | | | |
| Diphenylsiloxyphenyl trimethicone | 2.35 | 2.35 | 2.35 | 2.35 |
| Petrolatum, tocopherol | 1.55 | 1.55 | 1.55 | 1.55 |
| Diisostearyl malate | 0.75 | 0.75 | 0.75 | 0.75 |
| Mineral oil | 1.55 | 1.55 | 1.55 | 1.55 |
| Triethylhexanoin | 1.55 | 1.55 | 1.55 | 1.55 |
| Phenoxyethanol | 0.20 | 0.20 | 0.20 | 0.20 |
| Dibutylhydroxytoluene | 0.05 | 0.05 | 0.05 | 0.05 |
| Evaluation | | | | |
| SPF | 52 | 42 | 40 | 37 |
| UVAPF | 18 | 17 | 13 | 12 |

As shown in Table 7, the powder cosmetic of Example 12 had higher SPF and UVAPF values and excellent ultraviolet shielding performance compared to the cosmetics of Comparative Examples 19 to 21.

The present invention can be implemented in other forms than those described above without departing from the gist thereof. The embodiments disclosed in the present application are examples, and the present invention is not limited to these embodiments. The scope of the present invention is construed based on the claims, rather than the description above, and all modifications within the scope equivalent to the claims are included in the claims.

### INDUSTRIAL APPLICABILITY

The water-in-oil emulsion cosmetic, oil-in-water emulsion cosmetic, and powder cosmetic of the present invention can be applied to various cosmetics in which zinc oxide powder is blended (sunscreen, foundation, emulsion, skin cream, and the like). The zinc oxide powder of the present invention can constitute the oil-dispersible cosmetic composition, water-in-oil emulsion cosmetic, oil-in-water emulsion cosmetic, and powder cosmetic of the present invention, and the oil-dispersible cosmetic composition of the present invention can constitute the water-in-oil emulsion cosmetic and oil-in-water emulsion cosmetic of the present invention.

## Claims

1. A zinc oxide powder **characterized in that** D₅₀ of number distribution obtained from a transmission electron microscope image is 100 to 200 nm, the aspect ratio is 1.1 to 1.4, and the crystallite size obtained from an X-ray diffraction spectrum is 70 to 200 nm.

2. The zinc oxide powder according to Claim 1, wherein D₁₀ of number distribution obtained from a TEM image is 100 to 200 nm.

3. The zinc oxide powder according to Claim 1 or 2, wherein a ratio Tt550/Tt370, which is a ratio of a transmittance of light having a wavelength of 550 nm: Tt550 and a transmittance of light having a wavelength of 370 nm: Tt370 obtained using a spectral transmittance curve obtained from a film formed together with nitrocellulose, is 3.5 or more.

4. The zinc oxide powder according to Claim 3, wherein Tt370 is 24 or less.

5. The zinc oxide powder according to Claim 3 or 4, wherein Tt550 is 80 or more.

6. The zinc oxide powder according to any one of Claims 1 to 5, which is surface-treated.

7. The zinc oxide powder according to Claim 6, which is surface-treated with at least one surface treatment agent selected from the group consisting of silicone oil, fatty acid, and alkylsilane.

8. An oil-dispersible cosmetic composition **characterized in that** the zinc oxide powder according to any one of Claims 1 to 7 is dispersed in an oil.

9. A water-in-oil emulsion cosmetic **characterized in that** the oil-dispersible cosmetic composition according to Claim 8 is blended.

10. An oil-in-water emulsion cosmetic **characterized in that** the oil-dispersible cosmetic composition according to Claim 8 is blended.

11. A water-in-oil emulsion cosmetic **characterized in that** the zinc oxide powder according to any one of Claims 1 to 7 is blended.

12. An oil-in-water emulsion cosmetic **characterized in that** the zinc oxide powder according to any one of Claims 1 to 7 is blended.

13. A powder cosmetic **characterized in that** the zinc oxide powder according to any one of Claims 1 to 7 is blended.
